(19) Europäisches Patentamt
European Patent Office
Office européen des brevets

(11) Veröffentlichungsnummer: **0 091 057**
**B1**

# (12) EUROPÄISCHE PATENTSCHRIFT

(45) Veröffentlichungstag der Patentschrift: **11.06.86**

(51) Int. Cl.⁴: **C 07 D 223/20**, A 61 K 31/55

(21) Anmeldenummer: **83103054.9**

(22) Anmeldetag: **28.03.83**

(54) **5,6-Dihydro-11-alkylen-morphantridin-6-one, ihre Herstellung und diese enthaltende Arzneimittel.**

(30) Priorität: **06.04.82 DE 3212794**

(43) Veröffentlichungstag der Anmeldung: **12.10.83 Patentblatt 83/41**

(45) Bekanntmachung des Hinweises auf die Patenterteilung: **11.06.86 Patentblatt 86/24**

(84) Benannte Vertragsstaaten: **AT BE CH DE FR GB IT LI NL SE**

(56) Entgegenhaltungen:
**EP - A - 0 019 157**
**DE - A - 2 724 478**

**CHEMICAL ABSTRACTS, Band 70, Nr. 21, 26. Mai 1969, Seiten 332-333, Nr. 96656n, Columbus, Ohio, US**

(73) Patentinhaber: **BASF Aktiengesellschaft, Carl-Bosch-Strasse 38, D-6700 Ludwigshafen (DE)**

(72) Erfinder: **Steiner, Gerd, Dr., Oberer Waldweg 1, D-6719 Kirchheim (DE)**
Erfinder: **Friedrich, Ludwig, Dr., Nibelungenstrasse 8A, D-6831 Bruehl (DE)**
Erfinder: **Lenke, Dieter, Dr., Kekuleplatz 1, D-6700 Ludwigshafen (DE)**

**EP 0 091 057 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft 5,6-Dihydro-11-alkylen-morphantri-din-6-one, Verfahren zu ihrer Herstellung, und diese enthaltende therapeutische Mittel.

Es ist bekannt, daß tricyclische Ringsysteme vom Typ des 5,11-Dihydro-6H-pyrido[2.3-b][l.4]benzodiazepin-6-ons wertvolle Eigenschaften besitzen (Arzneim.-Forsch. 27, 356 (1977); DE-OS 27 24 501, DE-OS 27 24 478), die der Behandlung von Magen- und Duodenalulcera dienen.

Es wurde nun gefunden, daß 5,6-Dihydro-11-alkylen-morphan-tridin-6-one der Formel I

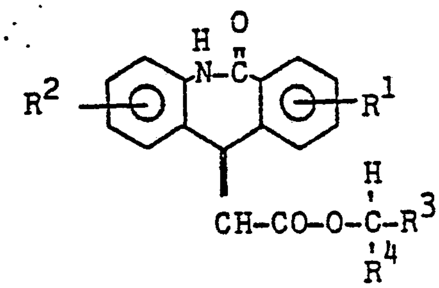

I

in der

$R^1$ und $R^2$ Wasserstoffatome, Halogenatome, Alkylreste mit 1 bis 3 C-Atomen oder Trifluormethylreste bedeuten,

$R^3$ einen Aminoalkylrest mit 1 bis 3 C-Atomen im Alkylrest, - wobei das Aminstickstoffatom durch 1 oder 2 Alkylreste mit mit 1 bis 3 C-Atomen substituiert oder Bestandteil eines 5- bis 7-gliedrigen gesättigten Ringes sein kann, der ein Stickstoff-, Sauerstoff- oder Schwefelatom als weiteres Heteroatom oder eine Carbonylgruppe enthalten kann, wobei ein vorhandenes Stickstoffatom durch einen Alkylrest mit 1 bis 3 C-Atomen, einen Hydroxyalkylrest mit 2 bis 3 C-Atomen oder durch eine Phenylgruppe, die durch Fluor, Chlor, Methoxy oder Methyl substituiert sein kann, substituiert sein kann -, bedeutet,

$R^4$ ein Wasserstoffatom darstellt oder

$R^3$ und $R^4$ zusammen einen 5- bis 7-gliedrigen gesättigten Ring darstellen, der durch einen oder mehrere Alkylreste mit 1 bis 3 C-Atomen substituiert sein kann oder durch eine intramolekulare Methylen- bzw. Bismethylen-Brücke in einen Bicyclus übergeführt sein kann und zusätzlich ein Stickstoffatom enthalten kann, wobei das zusätzlich vorhandene Stickstoffatom durch einen Alkylrest mit 1 bis 3 C-Atomen substituiert sein oder in Form des N-Oxids vorliegen kann,

sowie deren physiologisch verträglichen Säureadditionssalze wertvolle pharmakologische Eigenschaften besitzen.

Die erfindungsgemäßen Verbindungen der Formel I können als cis-trans-Isomere Ia und Ib vorliegen:

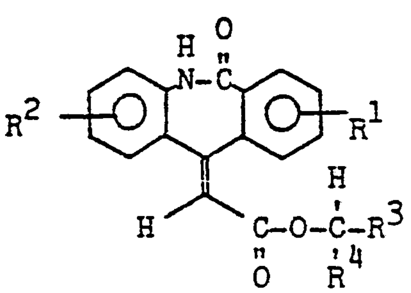

Ia, cis

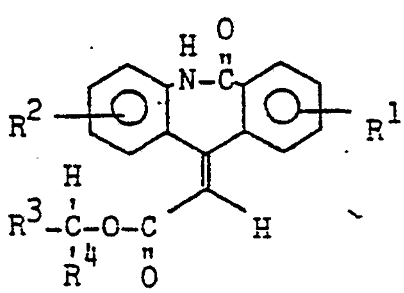

Ib, trans

Die Reste $R^1$ und $R^2$ sind vorzugsweise Wasserstoff, Chlor und Methyl.

Für den Rest $R^3$ seien speziell genannt: Aminomethyl, Methylaminomethyl, Dimethylaminomethyl 2-Dimethylaminoethyl, 4-Methylpiperazin-1-yl-methyl, Morpholin-1-yl-methyl, 3,5-Dimethyl-morpholin-1-yl-methyl, 2-Morpho-lin-1-yl-ethyl, 1-Methyl-morpholin-3-yl, 1-Benzyl-morpholin-3-yl, 1-Benzyl-morpholin-3-yl-6-on.

Für den Cyclus $R^3$ und $R^4$ seien hervorgehoben: 1-Methyl-piperidin-4-yl und N-Methyl-8-azabicyclo(3.2.l)octan-3-yl.

Besonders bevorzugt ist dabei für $R^3$ der Morpholin-1-yl-methyl-Rest.

Die folgenden Verbindungen sind als besonders wirksam zu nennen:
cis,trans-11-(2-Morpholin-1-yl)-ethoxycarbonylmethylen-5,6-dihydromorphantridin-6-on
cis-11-(2-Morpholin-1-yl)-ethoxycarbonylmethylen-5,6-dihydromorphantridin-6-on
trans-11-(2-Morpholin-1-yl)-ethoxycarbonylmethylen-5,6-di-hydromorphantridin-6-on
cis,trans-11-(2-Dimethylamino)-ethoxycarbonylmethylen-5,6-dihydromorphantridin-6-on
cis,trans-11-(3-Morpholin-1-yl)-propoxycarbonyl-methylen-5,6-dihydromorphantridin-6-on
Die erfindungsgemäßen Verbindungen der Formel I werden hergestellt, in dem man
a) eine Verbindung der Formel II

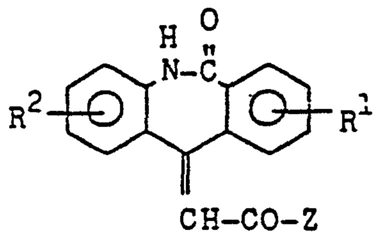

II

in der $R^1$ und $R^2$ die angegebenen Bedeutungen haben und Z eine nukleofuge Abgangsgruppe darstellt mit einem Aminoalkohol der Formel III
$HO\text{-}CHR^3R^4$ III,
worin $R^3$ und $R^4$ die angegebenen Bedeutungen haben, umsetzt oder
- falls $R^4$ ein Wasserstoffatom darstellt
b) eine Verbindung der Formel

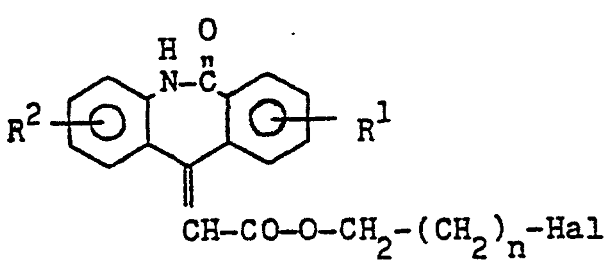

IV

worin Hal ein Halogenatom und n eine Zahl zwischen 1 und 3 bedeutet, mit dem entsprechenden Amin unter nukleophiler Substitution des Halogens umsetzt und die so erhaltene Verbindung gegebenenfalls in das reine cis- und trans-Isomere trennt und/oder gegebenenfalls in das Säureadditionssalz einer physiologisch verträglichen Säure überführt.

Als nukleofuge Abgangsgruppe für Z kommen Halogenatome, insbesondere Chlor, in Betracht.

Die Umsetzung a) erfolgt zweckmäßig in Gegenwart eines Moläquivalents eines tertiären Amins wie z.B. Triethylamin in einem inerten Lösungsmittel, wie einem cyclischen gesättigten Ether, insbesondere Tetrahydrofuran oder Dioxan, oder einem polaren aprotischen Lösungsmittel, vorzugsweise Dimethylformamid, bei Temperaturen von 0 bis 150° C, bevorzugt Raumtemperatur, und ist im allgemeinen innerhalb von 3 bis 10 Stunden beendet.

Gegebenenfalls kann auch in Gegenwart einer überschüssigen Menge des verwendeten Aminoalkohols III, der gleichzeitig als Lösungsmittel und gegebenenfalls als säurebindendes Mittel dient, gearbeitet werden.

Als nukleofuge Gruppen kommen in Betracht: Chlor, Brom, Iod und Alkylcarbonyloxy.

Die Reaktion b) wird in einer überschüssigen Menge des entsprechenden Amins, das gleichzeitig als Lösungsmittel dient, bei Temperaturen zwischen 50 und 150° C, vorzugsweise bei 110° C, oder in einem inerten organischen Lösungsmittel durchgeführt.

Die Überführung in die N-Oxide erfolgt in üblicher Weise, zweckmäßig mit wäßrigem Wasserstoffperoxid in ethanolischer Lösung. Die Überführung in das Säureadditionssalz einer physiologisch verträglichen Säure erfolgt ebenso in üblicher Weise.

Die Verbindungen der Formel I werden in der Regel in Form von Kristallen erhalten und können durch Umkristallisation aus den üblichen organischen Lösungsmitteln, bevorzugt aus einem niederen Alkohol, wie Ethanol, oder einem niedrigen Ester, vorzugsweise Essigsäurethylester, umkristallisiert oder durch Säulenchromatographie gereinigt werden.

Gegebenenfalls werden die erhaltenen erfindungsgemäßen Verbindungen in das Säureaddditionssalz einer physiologisch verträglichen Säure überführt. Als übliche physiologisch verträglich organische oder anorganische Säuren kommen beispielsweise in Betracht Salzsäure, Bromwasserstoffsäure,

Phosphorsäure oder Schwefelsäure sowie Oxalsäure, Maleinsäure, Fumarsäure, Milchsäure, Weinsäure, Äpfelsäure, Zitronensäure, Salicylsäure, Adipinsäure oder Benzoesäure. Weitere Säuren können beispielsweise dem J.Pharm.Sciences 66, 1 (1977) entnommen werden.

Die Säureadditionssalze werden in der Regel in an sich bekannter Weise durch Mischen der feien Base oder deren Lösungen mit der entsprechenden Säure oder deren Lösungen in einem organischen Lösungsmittel, beispielsweise einem niederen Alkohol wie Methanol, Ethanol oder Propanol oder einem niederen Keton wie Aceton, Methylethylketon oder Methylisobutylketon oder einem Ether wie Diethylether, Tetrahydrofuran oder Dioxan, erhalten. Zur besseren Kristallabscheidung können auch Mischungen der genannten Lösungsmittel verwendet werden.

Die Ausgangsverbindungen der Formel II werden erhalten, indem man ein cis,trans-11-Carboxymethylen-5,6-dihydro-morphantridin-6-on der Formel V

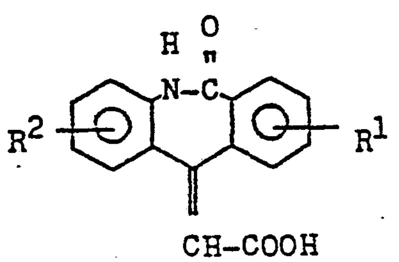

V

in der $R^1$ und $R^2$ die angegebenen Bedeutungen haben, in üblicher Weise mit überschüssigem Thionylchlorid bei Raumtemperatur in das Carbonsäurechlorid überführt.

Die Verbindungen der Formel V werden durch Verseifung der entsprechenden Ester (DE-OS 29 18 832) mit alkoholischer Alkalilauge bei 40 bis 90° C erhalten.

Die Verbindungen IV erhält man durch Umsetzung der Verbindungen II mit einem Halogenalkohol der Formel Hal-$(CH_2)_n$-OH, worin Hal ein Halogenatom und n eine Zahl zwischen 2 und 4 bedeutet. Die Umsetzung kann in einem inerten organischen Lösungsmittel oder einem Überschuß des Halogenalkohols bei 20 bis 100° C durchgeführt werden.

Zur Herstellung der reinen cis,trans-Isomeren geht man vorzugsweise von den cis- bzw. trans-Carbonsäure-Derivaten der Formel V aus. Die Isomerentrennung erfolgt am besten durch fraktionierte Kristallisation, die Zuordnung beruht auf den NMR-Signalen (270 MRz) des 11-ständigen Methylenprotons hei 6,12 bzw. 6,18 ppm.

Die Zugehörigkeit zur cis- oder trans-Reihe bei den einzeinen Isomeren erfolgt beispielsweise durch Röntgenstrukturanalyse.

Die erfindungsgemäßen Verbindungen und ihre physiologisch verträglichen Säureadditionssalze sind Pharmaka, die als wertvolle Heilmittel bei der Behandlung von Krankheitszuständen geeignet sind, die mit einer pathologisch gesteigerten Magensekretion einhergehen - wie z.B. Magen- und Duodenalulcera -.

Zur Untersuchung der antiulcerogenen Wirkung erhalten Gruppen von 10 weiblichen Sprague-Dawley Ratten (160 bis 180 g) 1 mg/kg Reserpin i.p. und bleiben danach 18 Stunden ohne Futter (Wasser ad libitum). Nach dieser Zeit erhalten die Tiere 21,5 mg/kg Indomethacin i.p. und die Prüfsubstanz per os und werden anschließend 6 Stunden lang bei einer Temperatur von 8° C gehalten und danach getötet. Die Mägen werden entnommen und die Fläche der ulcerösen Schleimhautläsionen bestimmt. Aus den linearen Regressionen zwischen den Logarithmen der applizierten Dosen und der relativen Verkleinerung der Fläche der Ulcerationen bez. auf die Kontrolltiere wird als ED 50 % die Dosis bestinmt, die eine Verkleinerung der Ulcusfläche um 50 % bewirkt.

Als Referenzsubstanz dient Pirenzepin (5,11-Dihydro-11-[(4methyl-1-piperazinyl)-acetyl]-6H-pyrido-[2,3,b][-1,4] benzodiazepin-6-on; DE-PS 1 795 183).

Die erfindungsgemäßen Verbindungen hemmen dosisabhängig die Bildung von Magenulcera (Tabelle 1).

Die Substanzen der Beispiele 3, 1, 2 und 14 übertreffen die Wirkung des bekannten Pharmakons Pirenzepin um den Paktor 10,2 bis 3,4.

**Tabelle 1**: Antiulcerogene Wirkung an der Ratte

| Bsp.Nr. | ED 50 % mg/kg | R.W. |
|---|---|---|
| 1, cis/trans | 1.90 | 3.4 |
| 1, cis | 1,50 | 4.3 |
| 1, trans | 5.30 | 1.2 |
| 2 | 0.64 | 10.2 |
| 14 | 0.83 | 7.8 |
| Pirenzepin | 6.5 | ≈1.0 |

Gegenstand der vorliegenden Erfindung sind daher weiter Arzneimittel, die eine Verbindung der Formel I oder deren physiologisch verträgliches Säureadditionssalz enthalten, sowie die Verwendung der neuen Verbindungen bei der Behandlung von Krankheitszuständen, die mit einer pathologisch gesteigerten Magensekretion einhergehen.

Die neuen Verbindungen können in den üblichen galenischen Applikationsformen, fest oder flüssig, angewendet werden, wie Tabletten, Filmtabletten, Kapseln, Pulver, Granulate, Dragees, Suppositorien oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln und/oder Antioxidantien verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 0,1 bis 99 Gew.%.

Die Dosierung der erfindungsgemäßen Verbindungen hängt vom Alter, Zustand und Gewicht des Patienten sowie von der Applikationsart ab. In der Regel beträgt die tägliche Wirkstoffdosis 5 bis 100, vorzugsweise 10 bis 80 mg.

Die vorliegende Erfindung wird durch die nachfolgenden Ausführungsbeispiele näher erläutert. Die Schmelzpunkte der cis,trans-Isomerengemische können dabei in Abhängigkeit vom cis-trans-Mengenverhältnis Schwankungen unterliegen.

**Beispiel 1** cis,trans-11-(2-Morpholin-1-yl)-ethoxycarbonylmethylen-5.6-dihydromorphantridin-6-on

a. Herstellung des Ausgangsproduktes

30,0 g (108 mM) 11-Carbomethoxymethylen-5,6-dihydro-morphantridin-6-on in 150 ml Ethanol wurden mit 200 ml 10 %iger Natronlauge versetzt und 2 h bei Rückflußtemperatur gerührt. Nach dem Abkühlen filtrierte man das Reaktionsgemisch und engte im Wasserstrahlvakuum auf etwa die Hälfte ein. Anschließend stellte man unter Eiskühlung mit konz. Salzsäure sauer und saugte die ausfallenden Kristalle unter gutem Nachwaschen mit Wasser ab. Man isolierte 27 g (94 %) cis,trans-11-Carboxymethylen-5,6-dihydromorphan-tridin-6-on, Schmp. 258-260° C.

31,0 g (124 mM) der so erstellten Verbindung wurden mit 200 ml Thionylchlorid versetzt und bei Raumtemperatur gerührt. Innerhalb 1 h trat Lösung ein. Nach weiterem einstündigem Rühren zog man das Thionylchlorid im Ölpumpenvakuum ab, nahm den Rückstand mit wenig Toluol auf und zog das Lösungsmittel wiederum vollständig ab. Das verbleibende cis,trans-11-Chlor-carbonylmethylen-5,6-dihydromorphantridin-6-on (Ausbeute 99 %) war genügend rein für die weitere Umsetzung.

b. Herstellung des Endproduktes

35,1 g (124 mM) 11-Chlorcarbonylmethylen-5,6-dihydro-morphantridin-6-on (cis,trans-Isomerengemisch) in 220 ml Dimethylformamid wurden unter gutem Rühren portionsweise mit 19,6 g (150 mM) N-(2-Hydroxyethyl)-morpholin und 12,5 g (124 mM) Triethylamin versetzt und 2 h unter Stickstoffatmosphäre bei Raumtemperatur gerührt. Nach Stehen über Nacht und vollständigem Abdestillieren des Lösungsmittels im Vakuum verteilte man den Rückstand zwischen Methylenchlorid und Wasser, machte die wäßrige Phase mit verdünnter Natronlauge schwach alkalisch, extrahierte die wäßrige Phase noch zweimal mit Methylenchlorid und wusch die vereinigten organischen Phasen mit Wasser gründlich nach. Trocknen und Einengen der organischen Phase lieferte 46 g Rohprodukt.

Zur Herstellung des reinen cis,trans-Isomerengemisches reinigte man das Rohprodukt durch Säulenchromatographie (Kieselgel, Methylenchlorid/Methanol 95/5). Man isolierte 31 g (66 %) farbloses cis,trans-11-(2-Morpholin-1-yl)-ethoxycarbonylmethylen-5,6-dihydro-morphantridin-6-on, Schmp. 92-94° C.

c. Isomerentrennung

Die Trennung in die cis,trans-Isomeren nimmt man am besten auf der Stufe des 11-Carboxymethylen-5;6-dihydromorphantridin-6-ons (vgl. a) vor:

20 g cis,trans-11-(2-Morpholin-1-yl)-ethoxycarbonyl-methylen-5,6-dihydro-morphantridin-6-on wurden in 1200 ml siedendem Methanol gelöst und nach Filtrieren fraktioniert kristallisiert. Man isolierte als am schwersten lösliche Fraktion 8,2 g farblose Kristalle, die nach Ausweis des 270 MHz NMR-Spektrums stark angereichertes

Isomer A (Methylen-H in 11-Posi-tion bei 6,18 ppm in $D_6$-DMSO) darstellen und durch nochmalige U,mkristallisation aus Methanol rein erhalten wurden, Schmp. 299-301°C (Zersetzung). Die Röntgenstrukturanalyse des Isomeren A bewies die trans-Anordnung der Carbonsäuregruppe in bezug auf die Carbonylgruppe der Säureamidstruktur im 7-gliedrigen Ring.

Aus der Mutterlauge kristallisierte eine Mischfraktion und danach als 3. oder 4. Fraktion 6,5 g des stark angereicherten Isomeren B (Methylen-H in 11-Position bei 6,12 ppm in $D_6$-DMSO) aus, das durch nochmalige Umkristallisation aus Methanol gereinigt wurde; Schmp. 297-299°C (Zersetzung).

Aus den so isolierten Isomeren A und B erhielt man dann analog a und b die reinen cis,trans-Isomeren des Endproduktes: aus 5,0 g des Isomeren A erhielt man nach Reinigung durch Säulenchromatographie (vgl. b) 3,6 g (51 %) 11-(2-Morpholin-1-yl)-ethoxycarbonylme-thylen-5,6-dihydromorphantridin-6-on mit trans-Konfiguration (Schmp. 88-90°C), während man aus 3,6 g des Isomeren B 2,7 g (53 %) 11-(2-Morpholin-1-yl)-ethoxycarbonylmethylen-5,6-dihydro-morphantridin-6-on mit cis-Konfiguration (Schmp. 98-100°C) erhielt.

Analog Beispiel 1 wurden hergestellt:

2. cis,trans-11-(2-Dimethylamino) -ethoxycarbonylmethylen-5,6-dihydromorphantridin-6-on. $H_2O$, Schmp. 78-80°C.

3. cis,trans-11-(3-Dimethylamino) -propoxycarbonylmethylen-5,6-dihydromorphantridin-6-on. 0,5 $H_2O$' Schmp. 74-76°C.

4. cis,trans-11-[2-(4-Methyl-piperazin-1-yl)] -ethoxycarbonylmethylen-5,6-dihydromorphantridin-6-on . 0,5 $H_2O$, Schmp. 87-90°C.

5. cis,trans-11-(1-Methyl-morpholin-3-yl) -methoxycarbonylmethylen-5,6-dihydromorphantridin-6-on, Schmp. 103-105°C.

6. cis,trans-11-(1-Benzyl-morpholin-3-yl) -methoxycarbonylmethylen-5,6-dihydromorphantridin-6-on. 0,5 $H_2O$' Schmp. 81-84°C.

7. cis,trans-11-(1-Benzyl-morpholin-3-yl-6-on)-methoxy-carbonylmethylen-5,6-dihydromorphantridin-6-on. 0,5 $H_2O$, Schmp. 91-94°C.

8. cis,trans-11-(1-Methyl-piperidin-4-yl)-oxycarbonyl-methylen-5,6-dihydromorphantridin-6-on, Schmp. 114-116°C.

9. cis,trans-11-(N-Methyl-8-azabicyclo(3.2.l)octan-3-yl-oxycarbonylmethylen-5,6-dihydromorphantridin-6-on. 1,5 $H_2O$, Schmp. 133-136°C (Säulenchromatographie: $CH_2Cl_2$/MeOH 70/30).

10. cis,trans-2-Chlor-11-(2-morpholin-1-yl) -ethoxycarbonylmethylen-5,6-dihydromorphantridin,6-on, Schmp. 88-90°C.

11. cis,trans-3-Methyl-11-(2-morpholin-1-yl)-ethoxy-carbonylmethylen-5,6-dihydromorphantridin-6-on, Schmp. 85-87°C.

**Beispiele 12** cis,trans-11-[2-(3,5-cis-Dimethyl-morpholin-1-yl)]-ethoxy-carbonylmethylen-5,6-dihydro-morphantridin-6-on

a. Herstellung des Ausgangsproduktes cis,trans-11-(2-Chlor)-ethoxycarbonylmethylen-5,6-dihydro-morphantridin-6-on

7,0 g (25 mM) 11-Chlorcarbonylmethylen-5,6-dihydro-morphantridin-6-on (cis,trans-Isomerengemisch) wurden portionsweise in 24 ml 2-Chlorethanol unter Rühren bei Raumtemperatur eingetragen (leicht exotherme Reaktion). Man ließ das Reaktionsgemisch noch 3 h weiterrühren und goß anschließend auf Eiswasser. Der halbkristalline Niederschlag wurde abgesaugt und gut mit Wasser ausgewaschen. Das Rohprodukt wurde anschließend in 500 ml Methylenchlorid gelöst und mit Wasser und 0,5 N Natronlauge gewaschen. Trocknen und Einengen der organischen Phase lieferte 7,6 g (92 %) Rohprodukt als Öl, das für die weitere Umsetzung genügend rein war.

b. Herstellung des Endproduktes

3,5 g (10,7 mM) cis,trans-11-(2-Chlor)-ethoxycarbonyl-methylen-5,6-dihydro-morphantridin-6-on wurden mit 8 ml cis-3,5-Dimethyl-morpholin 3 h bei 110°C gerührt. Nach dem Abkühlen löst man das dunkle Reaktionsgemisch in ca. 200 ml Methylenchlorid und wusch dreimal mit Wasser (pH-Wert der wäßrigen Phase wurde dabei auf 8 bis 9 gestellt) aus. Trocknen und Einengen der organischen Phase lieferte das Rohprodukt als dunkles Öl. Anschließend reinigt man das Rohprodukt durch Säulenchromatographie (Kieselgel, Laufmittel Methylenchlorid/Methanol 95/5). Man isolierte 2,4 g (55 %) farblose Kristalle, Schmp. 75-78°C.

Analog erhielt man:

13. cis,trans-11-[2-(3,5-trans-Dimethyl-morpholin-1-yl)]-ethoxycarbonylmethylen-5,6-dihydromorphantridin-6-on, Schmp. 67-69°C.

14. cis,trans-11-(3-Morpholin-1-yl)-propoxycarbonyl-methylen-5,6-dihydromorphantridin-6-on, Schmp.79-81°C.

15. cis,trans-11-(2-Methyl-morpholin-1-yl)-ethoxycarbonyl-methylen-5,6-dihydromorphantridin-6-on, Schmp. 76-78°C.

16. cis,trans-11-(Thiamorpholin-1-yl)-ethoxycarbonyl-methylen-5,6-dihydromorphantridin-6-on, Schmp. 83-85°C.

**Patentansprüche** für die Vertragsstaaten BE CH DE FR GB IT LI NL SE

1. 5,6-Dihydro-11-alkylen-morphantridin-6-one der Formel I

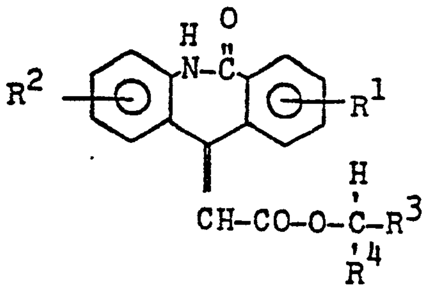

I

in der

$R^1$ und $R^2$ Wasserstoffatome, Halogenatome, Alkylreste mit 1 bis 3 C-Atomen oder Trifluormethylreste bedeuten,

$R^3$ einen Aminoalkylrest mit 1 bis 3 C-Atomen im Alkylrest, - wobei das Aminstickstoffatom durch 1 oder 2 Alkylreste mit 1 bis 3 C-Atomen substituiert oder Bestandteil eines 5- bis 7-glie-drigen gesättigten Ringes sein kann, der ein Stickstoff-, Sauerstoff- oder Schwefelatom als weiteres Heteroatom oder eine Carbonylgruppe enthalten kann, wobei ein vorhandenes Stickstoffatom durch einen Alkylrest mit 1 bis 3 C-Atomen, einen Hydroxyalkylrest mit 2 bis 3 C-Atomen oder durch eine Phenylgruppe, die durch Fluor, Chlor, Methoxy oder Methyl substituiert sein kann, substituiert sein kann -, bedeutet,

$R^4$ ein Wasserstoffatom darstellt oder

$R^3$ und $R^4$ zusammen einen 5- bis 7-gliedrigen gesättigten Ring darstellen, der durch einen oder mehrere Alkylreste mit 1 bis 3 C-Atomen substituiert sein kann oder durch eine intramolekulare Methylen- bzw. Bismethylen-Brücke in einen Bicyclus übergeführt sein kann und zusätzlich ein Stickstoffatom enthalten kann, wobei das zusätzlich vorhandene Stickstoffatom durch einen Alkylrest mit 1 bis 3 C-Atomen substituiert sein oder in Form des N-Oxids vorliegen kann,

sowie deren physiologisch verträglichen Säureadditionssalze.

2. Verbindungen der Formel I gemäß Anspruch 1, in der $R^1$ und $R^2$ Wasserstoffatome, Chloratome oder Methyl bedeuten, $R^3$ einen Aminoalkylrest mit 1 bis 3 C-Atomen - wobei das Aminstickstoffatom durch 1 oder 2 Alkylreste mit 1 bis 3 C-Atomen substituiert oder Bestandteil eines 5- bis 7-gliedrigen gesättigten Ringes sein kann, der ein Stickstoff- oder Sauerstoffatom als weiteres Heteroatom enthalten kann, wobei ein vorhandenes Stickstoffatom durch einen Alkylrest mit 1 bis 3 C-Atomen substituiert sein kann, bedeutet, $R^4$ ein Wasserstoffatom darstellt oder $R^3$ und $R^4$ zusammen einen 5- bis 7-gliedrigen gesättigten Ring darstellen, der durch einen oder mehrere Alkylreste mit 1 bis 3 C-Atomen substituiert sein kann oder durch eine intramolekulare Methylen- bzw. Bismethylen-Brücke in einen Bicyclus übergeführt ist und zusätzlich ein Stickstoffatom enthalten kann, wobei das zusätzlich vorhandene Stickstoffatom durch einen Alkylrest mit 1 bis 3 C-Atomen substituiert sein oder in Form des N-Oxides vorliegen kann, sowie deren physiologisch verträgliche Säureadditionssalze.

3. Verbindungen der Formel 1 gemäß Anspruch 1, in der $R^1$ und $R^2$ Wasserstoffatome, Chloratome oder Methyl bedeuten, $R^3$ einen Morpholin-1-yl-methyl- oder einen 2-Mor-pholin-1-yl-ethyl-Rest, wobei der Morpholin-Ring durch eine oder zwei Methylgruppen substituiert sein kann, oder einen Aminomethylrest, der an der Aminogruppe durch 1 oder 2 Methylgruppen substituiert sein kann, bedeutet und $R^4$ ein Wasserstoffatom darstellt, sowie deren physiologisch verträglichen Säureadditionssalze.

4. Verbindungen der Formel I gemäß Anspruch 1, 2 und 3 in der cis-Form.

5. Verbindungen der Formel I gemäß Anspruch 1, 2, 3 in der trans-Form.

6. cis,trans-11-(2-Morpholin-1-yl) -ethoxycarbonylmethylen-5,6-dihydromorphantridin-6-on.

7. cis-11-(2-Morpholin-1-yl)-ethoxycarbonylmethylen-5,6-dihydromorphantridin-6-on.

8. trans-11-(2-Morpholin-1-yl)-ethoxycarbonylmethylen-5,6-dihydromorphantridin-6-on.

9. cis,trans-11-(2-Dimethylamino)-ethoxycarbonylmethylen-5,6-dihydromorphantridin-6-on

10. cis,trans-11-(3-Morpholin-1-yl)-propoxycarbonyl-methylen-5,6-dihydromorphantridin-6-on.

11. Verbindungen der Formel I gemäß Anspruch 1 zur Bekämpfung von Ulcera.

**Patentansprüche** für den Vertragsstaat AT

1. Verfahren zur Herstellung von 5,6-Dihydro-11-alkylen-morphantridin-6-one der Formel I

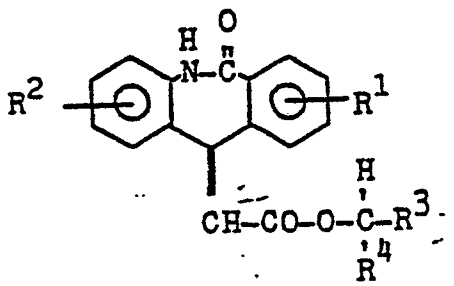

I

in der

$R^1$ und $R^2$ Wasserstoffatome, Halogenatome, Alkylreste mit 1 bis 3 C-Atomen oder Trifluormethylreste bedeuten,

$R^3$ einen Aminoalkylrest mit 1 bis 3 C-Atomen im Alkylrest, - wobei das Aminstickstoffatom durch 1 oder 2 Alkylreste mit 1 bis 3 C-Atomen substituiert oder Bestandteil eines 5- bis 7-gliedrigen gesättigten Ringes sein kann, der ein Stickstoff-, Sauerstoff- oder Schwefelatom als weiteres Heteroatom oder eine Carbonylgruppe enthalten kann, wobei ein vorhandenes Stickstoffatom durch einen Alkylrest mit 1 bis 3 C-Atomen, einen Hydroxyalkylrest mit 2 bis 3 C-Atomen oder durch eine Phenylgruppe, die durch Fluor, Chlor, Methoxy oder Methyl substituiert sein kann, substituiert sein kann -, bedeutet,

$R^4$ ein Wasserstoffatom darstellt oder

$R^3$ und $R^4$ zusammen einen 5- bis 7-gliedrigen gesättigten Ring darstellen, der durch einen oder mehrere Alkylreste mit 1 bis 3 C-Atomen substituiert sein kann oder durch eine intramolekulare Methylen- bzw. Bismethylen-Brücke in einen Bicyclus übergeführt sein kann und zusätzlich ein Stickstoffatom enthalten kann, wobei das zusätzlich vorhandene Stickstoffatom durch einen Alkylrest mit 1 bis 3 C-Atomen substituiert sein oder in Form des N-Oxids vorliegen kann, sowie deren physiologisch verträglichen Säureadditionssalze, <u>dadurch gekennzeichnet</u>, daß man

a) eine Verbindung der Formel II

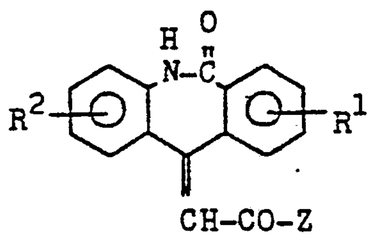

II

in der $R^1$ und $R^2$ die angegebenen Bedeutungen haben und Z eine nukleofuge Abgangsgruppe darstellt, mit einem Aminoalkohol der Formel III

$HC-CHR^3R^4$ III,

worin $R^3$ und $R^4$ die angegebene Bedeutung haben, umsetzt oder

b) - falls $R^4$ ein Wasserstoffatom darstellt

eine Verbindung der Formel IV

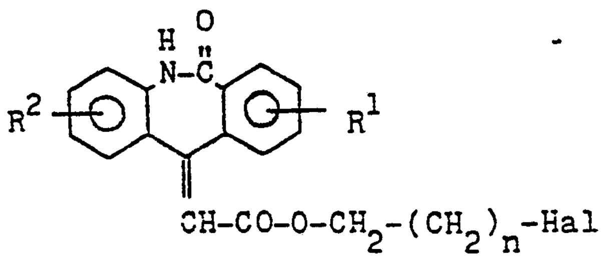

IV

worin Hal ein Halogenatom und n eine Zahl zwischen 1 und 3 bedeutet, mit dem entsprechenden Amin unter nukleophiler Substitution des Halogens umsetzt und die so erhaltene Verbindung gegebenenfalls in das reine cis- und trans-Isomere trennt und/oder gegebenenfalls in das Säureadditionssalz einer physiologisch verträglichen Säure überführt.

2. Verfahren gemäß Anspruch 1, dadurch gekennzeichnet, daß man Verbindungen der Formel I gemäß Anspruch 1, in der $R^1$ und $R^2$ Wasserstoffatome, Chloratome oder Methyl bedeuten, $R^3$ einen Aminoalkylrest mit 1 bis 3 C-Atomen - wobei das Aminstickstoffatom durch 1 oder 2 Alkylreste mit 1 bis 3 C-Atomen substituiert oder Bestandteil eines 5- bis 7-gliedrigen gesättigten Ringes sein kann, der ein Stickstoff- oder Sauerstoffatom als weiteres Heteroatom enthalten kann, wobei ein vorhandenes Stickstoffatom durch einen Alkylrest mit 1 bis 3 C-Atomen substituiert sein kann, bedeutet, $R^4$ ein Wasserstoffatom darstellt oder $R^3$ und $R^4$ zusammen einen 5- bis 7-gliedrigen gesättigten Ring darstellen, der durch einen oder mehrere Alkylreste mit 1 bis 3 C-Atomen substituiert sein kann oder durch eine intramolekulare Methylen- bzw. Bismethylen-Brücke in einen Bicyclus übergeführt ist und zusätzlich ein Stickstoffatom enthalten kann, wobei das zusätzlich vorhandene Stickstoffatom durch einen Alkylrest mit 1 bis 3 C-Atomen substituiert sein oder in Form des N-Oxides vorliegen kann, sowie deren physiologisch verträgliche Säureadditionssalze herstellt.

3. Verfahren gemäß Anspruch 2, dadurch gekennzeichnet, daß man Verbindungen der Formel I gemäß Anspruch 1, in der $R^1$ und $R^2$ Wasserstoffatome, Chloratome oder Methyl bedeuten, $R^3$ einen Morpholin-1-yl-methyl- oder einen 2-Morpholin-1-yl-ethyl-Rest, wobei der Morpholin-Ring durch eine oder zwei Methylgruppen substituiert sein kann, oder einen Aminomethylrest, der an der Aminogruppe durch 1 oder 2 Methylgruppen substituiert sein kann, bedeutet und $R^4$ ein Wasserstoffatom darstellt, sowie deren physiologisch verträglichen Säureadditionssalze herstellt.

4. Verfahren gemäß Anspruch 1, dadurch gekennzeichnet, daß man Verbindungen der Formel I gemäß Anspruch 1, 2 und 3 in der cis-Form herstellt.

5. Verfahren gemäß Anspruch 1, dadurch gekennzeichnet, daß man Verbindungen der Formel I gemäß Anspruch 1, 2, 3 in der trans-Form herstellt.

6. Verfahren gemäß Anspruch 1, dadurch gekennzeichnet, daß man cis,trans-11-(2-Morpholin-1-yl)-ethoxycarbonyl-methylen-5,6-dihydromorphantridin-6-on herstellt.

7. Verfahren gemäß Anspruch 1, dadurch gekennzeichnet, daß man cis-11-(2-Morpholin-1-yl) - ethoxycarbonylmethylen-5,6-dihydromorphantridin-6-on herstellt.

8. Verfahren gemäß Anspruch 1, dadurch gekennzeichnet, daß man trans-11-(2-Morpholin-1-yl)-ethoxycarbonyl-methylen-5,6-dihydromorphantridin-6-on herstellt.

9. Verfahren gemäß Anspruch 1, dadurch gekennzeichnet, daß man cis,trans-11-(3-Dimethylamino)-ethoxycarbonyl-methylen-5,6-dihydromorphantridin-6-on herstellt.

10. Verfahren gemäß Anspruch 1, dadurch gekennzeichnet, daß man cis,trans-11-(3-Morpholin-1-yl)-propoxy-carbonylmethylen-5,6-dihydromorphantridin-6-on herstellt.

**Claims** for the Contractding states: BE,CH,DE,FR,GB,IT,LI,NL,SE.

1. A 5,6-dihydro-11-alkylenemorphanthridin-6-one of the formula I

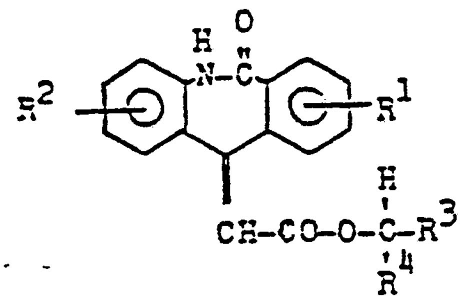

I,

where

$R^1$ and $R^2$ are each hydrogen, halogen, alkyl of 1 to 3 carbon atoms or trifluoromethyl,

$R^3$ is aminoalkyl, where alkyl is of 1 to 3 carbon atoms, and the amino nitrogen atom can be substituted by 1 or 2 alkyl radicals of 1 to 3 carbon atoms or can form part of a 5-membered, 6-membered or 7-membered saturated ring which can contain a nitrogen, oxygen or sulfur atom as a further hetero atom or can contain a carbonyl group, and any nitrogen atom present can be substituted by alkyl of 1 to 3 carbon atoms or hydroxyalkyl of 2 or 3 carbon atoms or by phenyl which may be substituted by fluorine, chlorine, methoxy or methyl, and

$R^4$ is hydrogen, or

$R^3$ and $R^4$ together form a 5-membered, 6-membered or 7-membered saturated ring which can be substituted by one or more alkyl radicals of 1 to 3 carbon atoms, or can be converted into a bicyclic structure by an intramolecular methylene or bismethylene bridge and can contain a further nitrogen atom which can be substituted by alkyl of 1 to 3 carbon atoms or can be present in the form of the N-oxide,

and its physiologically tolerated addition salts with acids.

2. A compound of the formula I as claimed in claim 1, where $R^1$ and $R^2$ are each hydrogen, chlorine or methyl, $R^3$ is aminoalkyl, where alkyl is of 1 to 3 carbon atoms and the amine nitrogen atom can be substituted by 1 or 2 alkyl radicals of 1 to 3 carbon atoms or can form part of a 5-membered, 6-membered or 7-membered saturated ring which can contain a nitrogen or oxygen atom as a further hetero atom, and any nitrogen atom present can be substituted by alkyl of 1 to 3 carbon atoms, and $R^4$ is hydrogen, or $R^3$ and $R^4$ together form a 5-membered, 6-membered or 7-membered saturated ring which can be substituted by one or more alkyl radicals of 1 to 3 carbon atoms, or is converted into a bicyclic structure by an intramolecular methylene or bismethylene bridge and may-contain a further nitrogen atom which can be substituted by alhyl of 1 to 3 carbon atoms or can be present in the form of the N-oxide, and its physiologically tolerated addition salts with acids.

3. A compound of the formula 1 as claimed in claim 1, where $R^1$ and $R^2$ are each hydrogen, chlorine or methyl, $R^3$ is morpholin-1-ylmethyl or 2-morpholin-1-ylethyl, where the morpholine ring can be monosubstituted or disubstituted by methyl, or $R^3$ is aminomethyl which can be substituted at the amino group by 1 or 2 methyl groups, and $R^4$ is hydrogen, and its physiologically tolerated addition salts with acids.

4. A compound of the formula 1 as claimed in claims 1, 2 and 3, in the cis-form.

5. A compound of the formula 1 as claimed in claims 1, 2 and 3, in the trans-form.

6. cis,trans-11-(2-Morpholin-1-yl)-ethoxycarbonylmethylene-5,6-dihydromorphanthridin-6-one.

7. cis-11-(2-Morpholin-1-yl)-ethoxycarbonylmethylene-5,6-dihydromorphanthridin-6-one.

8. trans-11-(2-Morpholin-1-yl)-ethoxycarbonylmethylene-5,6-dihydromorphanthridin-6-one.

9. cis,trans-11-(2-Dimethylamino)-ethoxycarbonylmethylene-5,6-dihydromorphanthridin-6-one.

10. cis,trans-11-(3-morpholin-1-yl)-propoxycarbonylmethylene-5,6-dihydromorphanthridin-6-one.

11. A compound of the formula I as claimed in claim 1 for treating ulcers.

**Claims** for the Contracting state : AT.

1. A process for the preparation of a 5,6-dihydro 11 alkylenemorphanthridin-6-one of the formula I

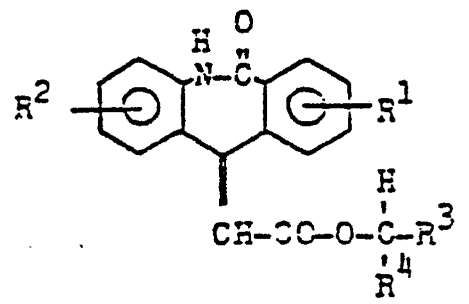

I ,

where

$R^1$ and $R^2$ are each hydrogen, halogen, alkyl of 1 to 3 carbon atoms or trifluoromethyl,

$R^3$ is aminoalkyl, where alkyl is of 1 to 3 carbon atoms, and the amino nitrogen atom can be substituted by 1 or 2 alkyl radicals of 1 to 3 carbon atoms or can form part of a 5-membered, 6-membered or 7-membered saturated ring which can contain a nitrogen, oxygen or sulfur atom as a further hetero atom or can contain a carbonyl group, and any nitrogen atom present can be substituted by alkyl of 1 to 3 carbon atoms or hydroxyalkyl of 2 or 3 carbon atoms or by phenyl which may be substituted by fluorine, chlorine, methoxy or methyl, and

$R^4$ is hydrogen, or

$R^3$ and $R^4$ together form a 5-membered, 6-membered or 7-membered saturated ring which can be substituted by one or more alkyl radicals of 1 to 3 carbon atoms, or can be converted into a bicyclic structure by an intramolecular methylene or bismethylene bridge and can contain a further nitrogen atom which can be substituted by alkyl of 1 to 3 carbon atoms or can be present in the form of the N-oxide, and its physiologically tolerated addition salts with acids, wherein

a) a compound of the formula II

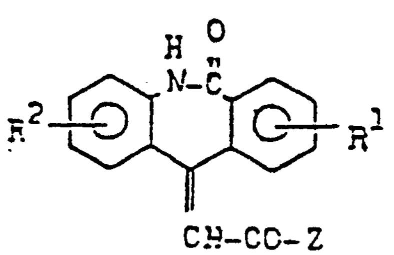

II,

where $R^1$ and $R^2$ have the above meanings and Z is a nucleofugic leaving group, is reacted with an aminoalcohol of the formula III

$HO\text{-}CHR^3R^4$ III

where $R^3$ and $R^4$ have the above meanings, or b) where $R^4$ is hydrogen, a compound of the formula IV

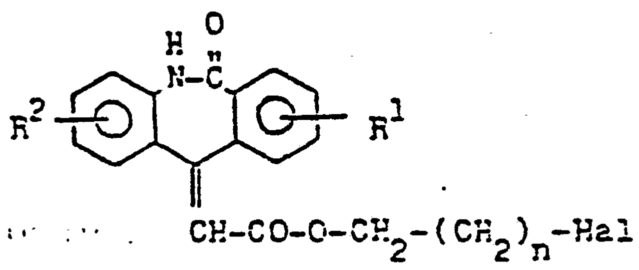

IV,

where Hal is halogen and n is 1, 2 or 3, is reacted with an appropriate amine, with nucleophilic substitution of the halogen,

and, if desired, the compound thus obtained is separated into the pure cis and trans isomers and/or, if desired, converted into an addition salt with a physiologically tolerated acid.

2. A process as claimed in claim 1, wherein a compound of the formula I as claimed in claim 1, whee $R^1$ and $R^2$ are each hydrogen, chlorine or methyl, $R^3$ is aminoalkyl, where alkyl is of 1 to 3 carbon atoms and the amine nitrogen atom can be substituted by 1 or 2 alkyl radicals of 1 to 3 carbon atoms or can form part of a 5-membered, 6-membered or 7-membered saturated ring which can contain a nitrogen or oxygen atom as a further hetero atom, and any nitrogen atom present can be substituted by alkyl of 1 to 3 carbon atoms, and $R^4$ is hydrogen, or $R^3$ and $R^4$ together form a 5-membered, 6-membered or 7-membered saturated ring which can be substituted by one or more alkyl radicals of 1 to 3 carbon atoms, or is converted into a bicyclic structure by an intramolecular methylene or bismethylene bridge and may contain a further nitrogen atom which can be substituted by alkyl of 1 to 3 carbon atoms or can be present in the form of the N-oxide, or its physiologically tolerated addition salts with acids, are prepared.

3. A process as claimed in claim 2, wherein a compound of the formula I as claimed in claim 1, where $R^1$ and $R^2$ are each hydrogen, chlorine or methyl, $R^3$ is morpholin-1-ylmethyl or 2-morpholin-1-ylethyl, where the morpholine ring can be monosubstituted or disubstituted by methyl, or $R^3$ is aminomethyl which can be substituted at the amino group by 1 or 2 methyl groups, and $R^4$ is hydrogen, and its physiologically tolerated addition salts with acids, are prepared.

4. A process as claimed in claim 1, wherein a compound of the formula I as claimed in claims 1, 2 and 3, in the cis-form, is prepared.

5. A process as claimed in claim 1, wherein a compound of the formula I as claimed in claims 1, 2 and 3, in the trans-form, is prepared.

6. A process as claimed in claim 1, wherein cis,trans-11-(2-morpholin-1-yl)-ethoxycarbonylmethylene-5,6-dihydro-morphanthridin-6-one is prepared.

7. A process as claimed in claim 1, wherein cis-11-(2-morpholin-1-yl)-ethoxycarbonylmethylene-5,6-dihydro-morphanthridin-6-one is prepared.

8. A process as claimed in claim 1, wherein trans-11-(2-morpholin-1-yl)-ethoxycarbonylmethylene-5,6-dihydro-morphanthridin-6-one is prepared.

9. A process as claimed in claim 1, wherein cis,trans-11-(3-dimethylamino)-ethoxycarbonylmethylene-5,6-dihydro-morphanthridin-6-one is prepared.

10. A process as claimed in claim 1, wherein cis,trans-11-(3-morpholin-1-yl)-propoxycarbonylmethylene-5,6-dihydro-morphanthridin-6-one is prepared.

**Revendications** pour les Etats contractants:BE,CH,DE,FR,IT,LI,NL,SE.

1. 5,6-Dihydro-11-alkylène-morphantridine-6-ones de la formule I

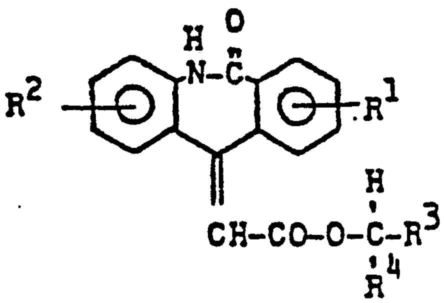

(I),

dans laquelle

$R^1$ et $R^2$ désignent des atomes d'hydrogène ou d'halogène, des radicaux alkyle en $C_1$ à $C_3$ ou des groupes trifluorométhyle,

$R^3$ désigne un groupe amino-alkyle à radical alkyle en $C_1$ à $C_3$, l'atome d'azote de la fonction amino pouvant être substitué par un radical ou deux radicaux alkyle en $C_1$ à $C_3$ ou faire partie d'un noyau penta- à heptagonal sature pouvant comporter un deuxième hétéro-atome azote, oxygène ou soufre ou un groupe carbonyle, l'atome d' azote éventuel pouvant être substitué par un radical alkyle en $C_1$ à $C_3$, un radical hydroxy-alkyle en $C_2$ ou $C_3$ ou un groupe phényle éventuellement fluoro-, chloro-, méthoxy- ou méthyl-substitué,

$R^4$ représente un atome d'hydrogène ou

$R^3$ et $R^4$ forment ensemble un noyau penta- à heptagonal saturé pouvant être substitug par un plusieurs radicaux alkyle en $C_1$ à $C_3$ ou constituer un bicycle par l'intermédiaire d'un pont méthylène ou bis-méthylène intramoléculaire et comporter un atome d'azote supplémentaire éventuellement substitué par un radical alkyle en $C_1$ à $C_3$ ou former un N-oxyde,

ainsi que les sels d'addition d'acides physiologiquement acceptables de ces composés.

2. Composés de la formule I selon la revendication 1, pour lesquels $R^1$ et $R^2$ désignent des atomes d'hydrogène ou de chlore ou des radicaux méthyle; $R^3$ désigne un groupe amino-alkyle en $C_1$ à $C_3$, l'atome d'azote de la fonction amino pouvant être substitué par un ou deux radicaur alkyle en $C_1$ à $C_3$ ou faire partie d'un noyau penta- à heptagonal saturé, pouvant comporter un deuxième hétéroatome azote ou oxygène, l'atome d'azote éventuel pouvant être substitué par un radical alkyle en $C_1$ à $C_3$; $R^4$ est un atome d'hydrogène ou $R^3$ et $R^4$ forment ensemble un noyau penta- à heptagonal saturé, pouvant être substitué par un ou plusieurs radicaux alkyle en $C_1$ à $C_3$ ou constituer un bicycle par l'intermédiaire d'un pont méthylène ou bis-méthylène intramoléculaire et comporter un atome d' azote supplémentaire éventuellement substitué par un radical alkyle en $C_1$ à $C_3$ ou former un N-oxyde, ainsi que leurs sels d'addition d'acides physiologiqument acceptables.

3. Composés de la formule I selon la revendication 1, pour lesquels $R^1$ et $R^2$ désignent des atomes d'hydrogene ou de chlore ou des radicaux méthyle; $R^3$ désigne un groupe morpholin-1-yl-méthyle ou 2-morpholin-1-yl-éthyle, le noyau morpholinique pouvant être substitué par un ou deux radicaux méthyle, ou un groupe aminométhyle, dont le groupe amino peut être substitué par un ou deux radicaux méthyle, et $R^4$ est un atome d'hydrogène, ainsi que leurs sels d'addition d'acides physiologiquement acceptables.

4. Composés de la formule I selon la revendication 1, 2 ou 3 dans leur forme cis.

5. Composés de la formule I selon la revendication 1, 2 ou 3 dans leur forme trans.

6. La 11-(2-morpholin-1-yl)-éthoxycarbonyl-méthylène-5,6-dihydro-morphantridine-6-one cis, trans.

7. La 11-(2-morpholin-1-yl)-éthoxycarbonyl-methylène-5,6-dihydro-morphantridine-6-one cis.

8. La 11-(2-morpholin-1-yl)-éthoxycarbonyl-methylène-5,6-dihydro-morphantridine-6-one trans.

9. La 11-(2-diméthylamino)-éthoxycarbonyl-méthylène-5,6-dihydro-morphantridine-6-one cis, trans.

10. La 11-(3-morpholin-1-yl)-propoxycarbonyl-méthylène-5,6-dihydro-morphantridine-6-one cis, trans.

11. Composés de la formule I selon la revendication 1 pour le traitement d'ulcères.

**Revendications** pour l'Etat contractant: AT.

1. Procédé de préparation de 5,6-dihydro-11-alkylène-morphantridine-6-ones de la formule I

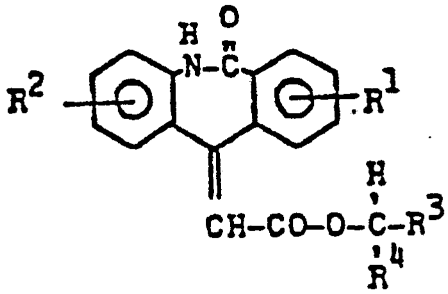

(I),

dans laquelle

$R^1$ et $R^2$ désignent des atomes d'hydrogène ou d'halogène, des radicaux alkyle en $C_1$ à $C_3$ ou des groupes trifluorométhyle,

$R^3$ désigne un groupe amino-alkyle à radical alkyle en $C_1$ à $C_3$, l'atome d'azote de la fonction amino pouvant être substitué par un radical ou deux radicaux alkyle en $C_1$ à $C_3$ ou faire partie d'un noyau penta- à heptagonal saturg pouvant comporter un deuxième hétéro-atome azote, oxygène ou soufre ou un groupe carbonyle, l'atome d' azote éventuel pouvant être substitué par un radical alkyle en $C_1$ à $C_3$, un radical hydroxy-alkyle en $C_2$ ou $C_3$ ou un groupe phényle éventuellement fluoro-, chloro-, mgthoxy- ou méthyl-substitué,

$R^4$ représente un atome d'hydrogène ou

$R^3$ et $R^4$ forment ensemble un noyau penta- à heptagonal saturé pouvant être substitué par un plusieurs radicaux alkyle en $C_1$ à $C_3$ ou constituer un bicycle par l'intermédiaire d'un pont méthylène ou bis-méthylène intramoléculaire et comporter un atome d'azote supplémentaire éventuellement substitué par un radical alkyle en $C_1$ à $C_3$ ou former un N-oxyde,

ainsi que des sels d'addition d'acides physiologiquement acceptables de ces composés, caractérisé en ce que l'on fait réagir :

a) un composé de la formule II

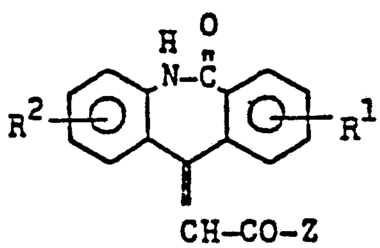

(II),

dans laquelle $R^1$ et $R^2$ possèdent les significations définies et Z représente un groupe de clivage nucléofuge, avec un amino-alcool de la formule III

HO - $CHR^3R^4$ (III),

dans laquelle $R^3$ et $R^4$ possèdent la signification définie ou

b) - si $R^4$ = H -

un composé de la formule IV

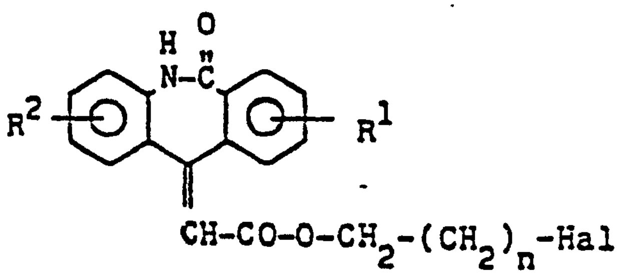

(IV),

dans laquelle Hal désigne un atome d'halogène et n est un nombre valant entre 1 et 3, avec une amine appropriée avec substitution nucléophile de l'halogène, le composé obtenu pouvant éventuellement être séparé en ses isomères cis et trans purs et(ou) éventuellement transformé en sel d'addition d'un acide physiologiquement acceptable.

2. Procédé suivant la revendication 1, caractérisé en ce qu'il est mis en oeuvre pour préparer des composés de la formule I selon la revendication 1, pour lesquels $R^1$ et $R^2$ désignent des atomes d'hydrogène ou des atomes de chlore ou des radicaux méthyle; $R^3$ représente un groupe amino-alkyle en $C_1$ à $C_3$, l'atome d'azote de la fonction amino pouvant être substitué par un ou deux radicaux alkyle en $C_1$ à $C_3$ ou faire partie d'un noyau pentaà heptagonal saturé, pouvant comporter un deuxième hétéroatome azote ou oxygène, l'atome d'azote éventuel pouvant être substitué par un radical alkyle en $C_1$ à $C_3$; $R^4$ est un atome d'hydrogène ou $R^3$ et $R^4$ forment ensemble un noyau penta- à heptagonal saturé, pouvant être substitué par un ou plusieurs radiçaux alkyle en $C_1$ à $C_3$ ou constituer un bicycle par l'intermédiaire d'un pont méthylène ou bis-méthylène intramoléculaire et comporter un atome d' azote supplémentaire éventuellement substitué par un radical alkyle en $C_1$ à $C_3$ ou former un N-oxyde, ainsi que leurs sels d'addition d'acides physiologiqument acceptables.

3. Procédé suivant la revendication 2, caractérisé en ce qu'il est mis en oeuvre pour préparer des composés de la formule I selon la revendication 1, pour lesquels $R^1$ et $R^2$ désignent des atomes d'hydrogène ou des atomes de chlore ou des radicaux méthyle; $R^3$ représente un groupe morpholin-1-yl-méthyle ou 2-morpholin-1-yl-éthyle, le noyau morpholinique pouvant être substitué par un ou deux radicaux méthyle, ou un groupe amino-méthyle, dont le groupe amino peut être substitué par un ou deux radicaux méthyle, et $R^4$ est un atome d'hydrogène, ainsi que leurs sels d'addition d'acides physiologiquement acceptables.

4. Procédé suivant la revendication 1, caractérisé en ce qu'il sert à préparer des composés de la formule I selon la revendication 1, 2 ou 3 dans leur forme cis.

5. Procédé suivant la revendication 1, caractérisé en ce qu'il sert à préparer des composés de la formule I selon la revendication 1, 2 ou 3 dans leur forme trans.

6. Procédé suivant la revendication 1, caractérisé en ce que l'on prépare la 11-(2-morpholin-1-yl)-éthoxycarbonyl-méthylène-5,6-dihydro-morphantridine-6-one cis, trans.

7. Procédé suivant la revendication 1, caractérisé en ce que l'on prépare la 11-(2-morpholin-1-yl)-éthoxycarbonyl-méthylène-5,6-dihydro-morphantridine-6-one cis.

8. Procédé suivant la revendication 1, caractérisé en ce que l'on prépare la 11-(2-morpholin-1-yl)-éthoxycarbonyl-méthylène-5,6-dihydro-morphantridine-6-one trans.

9. Procédé suivant la revendication 1, caractérisé en ce que l'on prépare la 11-(3-diméthylamino)-éthoxycarbonyl-méthylène-5,6-dihydro-morphantridine-6-one cis, trans.

10. Procédé suivant la revendication 1, caractérisé en ce que l'on prépare la 11-(3-morpholin-1-yl)-propoxycarbonyl-méthylène-5,6-dihydro-morphantridine-6-one cis, trans.